Europäisches Patentamt

**European Patent Office**

Office européen des brevets

(19)

(11) Publication number: **0 182 521**
A2

(12) # EUROPEAN PATENT APPLICATION

(21) Application number: **85307774.1**

(51) Int. Cl.⁴: **C 07 C 51/50,** C 07 C 85/26,
C 07 C 87/52, C 07 C 91/06,
C 07 C 101/26, C 07 C 89/04,
C 07 C 99/00

(22) Date of filing: **28.10.85**

(30) Priority: **22.11.84 GB 8429529**

(43) Date of publication of application: **28.05.86**
**Bulletin 86/22**

(84) Designated Contracting States: **AT BE DE FR GB IT NL
SE**

(71) Applicant: **MORTON THIOKOL, INC., 110 North Wacker
Drive, Chicago Illinois 60606 (US)**

(72) Inventor: **Sanglet, Phillipe R., Lange Violette Straat 114,
B-9500 Gent (BE)**

(74) Representative: **Bankes, Stephen C. D. et al, Baron &
Warren 18 South End Kensington, London W8 5BU (GB)**

(54) **Process for stabilizing organic compounds and compositions stabilized thereby.**

(57) Organic compounds are stabilized, in particular against oxidative degradation, by the addition of sodium borohydride and a transition metal salt, preferably a group VIII metal salt such as nickel chloride. The invention is particularly applicable to amines and fatty acids. The stabilizers are found to have a remarkable synergistic effect in maintaining stability and colour of an amine, which is enhanced by distillation of the amine.

# PROCESS FOR STABILIZING ORGANIC COMPOUNDS AND COMPOSITIONS STABILIZED THEREBY

This invention relates to the stabilization and purification of organic compounds.

It is well known that some organic compounds, particularly amines, are subject to oxidative degradation under conditions of heat or exposure to light (particularly ultra violet light), air or metal cations. Such oxidation imparts a red, yellow or brown colour to an amine. This effect can be mitigated to some extent by distilling the amine and protecting it against such conditions, but is always liable to re-occur.

It has been proposed, for example in JP-A-59-25355, to stabilize amines against such discolouration by the addition of a small amount of sodium borohydride, $NaBH_4$. This acts as a strong reducing agent and reacts virtually quantitatively with a wide variety of oxidised impurities which contribute to the colouring of the amines. For example, aldehydes and ketones are reduced to alcohols, Schiff's bases are reduced to secondary amines, alkyl peroxides are reduced to alkanol and metal cations are reduced to the respective metals.

The use of $NaBH_4$ (hereinafter referred to as SBH) can greatly improve the stability of organic compounds such as amines and fatty acids, but for some purposes there is a demand for considerable further improvement in stability and colour.

For example, crude ethylene diamine tetraacetic acid (EDTA) when first produced generally has an APHA colour rating of 700-800. Treatment with peroxide and activated carbon can reduce this to 300-400, and further carbon treatment can reduce the level to 200 APHA. SBH can reduce the APHA colour still further, but not to the much lower levels such as 20-60 APHA required in some markets.

According to one aspect of the present invention, an organic compound is stabilized with sodium borohydride and a transition metal salt.

The transition metal salt is preferably a salt of a group VIII metal, most preferably nickel or cobalt. The salt may suitably be a halide, preferably chloride, or an organic salt such as an acetate.

The combination of SBH and the transition metal salt can be used to stabilize a wide range of both aliphatic and aromatic compounds, for example aniline, alkanolamines and ethylene amines, and fatty acids such as oleic acid. The stabilizers are particularly effective in the case of relatively short chain amines such as ethylene diamine tetraacetic acid (EDTA). A remarkable synergistic effect has been found using small quantities of both stabilizing components. The effect is particularly marked if combined with distillation of the organic compound.

While the precise mechanism is uncertain, one possibility is that hydrogen is generated in situ and acts in conjunction with a metal salt such as nickel chloride $NiCl_2$ to reduce unsaturated groups by catalytic hydrogenation.

The amounts of stabilizing components used are preferably in ranges of 10 to 1000 ppm by weight for the SBH and 5 to 500 ppm by weight for the metal salt based in each case on the organic compound to be stabilized.

Preferred embodiments of the invention will be further illustrated in the following examples, wherein all proportions and percentages given are by weight unless otherwise specified.

EXAMPLE 1

To one litre of 2-(ethylamino)ethanol (also known as ethylamino - ethanolamine EAEA) were added 200 ppm of $NiCl_2.6H_2O$ and 500 ppm of SBH as a stabilized aqeuous solution. The mixture was stirred for 30 mins. at room temperature and distilled under vacuum. The results were as follows:

|                    | APHA Colour |
|--------------------|-------------|
| Untreated EAEA     | 350 - 400   |
| First 10% fraction:| 25          |
| Next 85% fraction: | < 5         |

Distilling EAEA under vacuum without SBH and the nickel salt gave an APHA value of 80-100.

EXAMPLE 2

The process of Example 1 was repeated for EDTA. The resulting APHA value was approximately 15. Precipitating EDTA without SBH and the nicket salt gave an APHA value of 60-70.

EXAMPLE 3

Tests were carried out on a commercial sample of EDTA. The EDTA, having an initial APHA value of 300, was acidified to pH 1.5 with a 30% HCl solution, washed twice with water and dried to give a powder. The powder was dissolved in 30% NaOH and the colour of the solution determined giving an APHA value of 80.

The solution was then acidified to pH 5.5-6 with a 30% HCl solution and 400 ppm of SBH (as a 1/5 dilution of a stabilized aqeuous solution) was added. This corresponds to about 1000ppm based on the acid. A trace of $Nicl_2$ was added and the solution was acidified to pH2. The precipitate was then filtered off and redissolved in 30% NaOH to give a 40 EDTA solution. The colour of this solution was found to be 10-20 APHA.

When the above process was carried out using 200, 500 and 1000 ppm SBH, based on the EDTA solution, but without addition of nickel chloride, no adequate reduction in colour was obtained.

## EXAMPLE 4

While agitating and under a nitrogen atmosphere, 0.02% $NiCl_2.6H_2O$ and 0.05% $NaBH_4$ powder were added to a round bottom flask containing 75g of oleic acid. The flask was then attached to a distillation apparatus and vacuum distilled at 10-12mm of Hg.

A control without any additives, as well as one using just 0.05% $NaBH_4$ were also prepared for comparison.

A summary of the process time-temperature and the properties of the resulting oleic acid is set out in Table 1.

### TABLE 1

| Mode of Purification | Initial Colour(Pt/Co) | After 3 wk @ 51°C under $N_2$ | |
| --- | --- | --- | --- |
| | | Carbonyl1 (ppm) | Colour Gardner |
| Vac. dist. only | 60 | 845 | 4 |
| $NaBH_4$/1h agit.at amb./ 1h agit.at 68°C/Vac.dist. | 60 | 860 | 3 |
| $NaBH_4$ & $NiCl_2.6H_2O$/ vac.dist. | 90 | 707 | <2 |

1 As C==O M.W. 28

## EXAMPLE 5

To 75g N,N, dimethylcocoamine was added 0.02% $NiCl_2$ $6H_2O$ and 0.05% $NaBH_4$ powder. Mixture was allowed to agitate for 1.0 hour at ambient temperature under nitrogen and then vacuum distilled at 10-12mm Hg.

Controls, one without any additives and another using 0.05% $NaBH_4$ only were processes in a similar manner.

A summary of the properties of the resulting amine is set out in Table 2.

0182521

TABLE 2

| Mode of Purification | Initial Colour(Visual) | After 3 wk @ 51°C under $N_2$ | |
| --- | --- | --- | --- |
| | | Carbonyl (ppm) | Colour (Visual) |
| Dist.only | $H_2O$ white | 648 | $H_2O$ white |
| $NaBH_4$ | $H_2O$ white | 445 | sl.yellow tint |
| $NaBH_4/NiCl_2 \cdot 6H_2O$ | $H_2O$ white | 392 | $H_2O$ white |

1 As C==O M.W. 28

## CLAIMS

1. A process for stabilising an organic compound wherein sodium borohydride (SBH) is added to the said compound, characterised in that there is also added to the organic compound a transition metal salt.

2. A process according to claim 1, characterised in that the transition metal salt is a halide.

3. A process according to claim 1 or claim 2, characterised in that the transition metal salt is a salt of a metal in group VIII of the periodic table.

4. A process according to claim 3, characterised in that the transition metal salt is a nickel or cobalt salt.

5. A process according to claim 4, characterised in that the transition metal salt is nickel chloride.

6. A process according to any preceding claim, characterised in that the organic compound is a fatty acid or an amine.

7. A process according to claim 6, characterised in that the organic compound is aniline, 2-(ethylamino) ethanol.(EAEA) or ethylene diamine tetraacetic acid (EDTA).

8. A process according to any preceding claim, characterised in that the proportion of SBH added is from 10 to 1000 ppm by weight based on the organic compound.

9. A process according to any preceding claim, characterised in that the proportion of transition metal salt added is from 5 to 500 ppm by weight based on the organic compound.

10. A process according to any preceding claim, characterised in that the organic compound containing the stabilizing components is distilled.

11. A composition comprising an organic compound stabilized with sodium borohydride (SBH) characterised in that it also contains a stabilizing amount of a transition metal salt.

12. A composition according to claim 11, characterised in that the transition metal salt is a halide.

13. A composition according to claim 11 or claim 12, characterised in that the transition metal salt is a salt of a metal in group VIII of the periodic table.

14. A composition according to claim 13, characterised in that the transition metal salts is a nickel or cobalt salt.

15. A composition according to claim 14, characterised in that the transition metal salt is nickel chloride.

16. A composition according to any one of claims 11 to 15, characterised in that the organic compound is an amine or a fatty acid.

17. a composition according to claim 16, characterised in that the organic compound is aniline, 2-(ethylamino) ethanol(EAEA) or ethylene diamine tetraacetic acid (EDTA).

18. A composition according to any one of claims 11 to 17, characterised in that it contains 10-1000 ppm by weight of SBH based on the organic compound.

19. A composition according to any one of claims 11 to 18, characterised in that it contains 5 to 500 ppm by weight of the transition metal salt, based on the organic compound.